# EUROPEAN PATENT APPLICATION

(11) **EP 4 642 138 A1**
(43) Date of publication of application: **29.10.2025**
(21) Application number: 24857788.4
(22) Date of filing: 15.04.2024
(51) Int. Cl.: H04W 72/542

(54) **NON-CONTACT SENSING METHOD AND DEVICE**

(30) Priority: 30.08.2023 CN 202311112774
(71) Applicant: Huawei Technologies Co., Ltd., Shenzhen, Guangdong 518129 (CN)
(72) Inventor: CHENG, Xingqing, Shenzhen, Guangdong 518129 (CN); QUAN, Chao, Shenzhen, Guangdong 518129 (CN); GAO, Lei, Shenzhen, Guangdong 518129 (CN); LI, Dejian, Shenzhen, Guangdong 518129 (CN); YANG, Changqing, Shenzhen, Guangdong 518129 (CN)
(74) Representative: Körber, Martin Hans
(86) International application number: PCT/CN2024/087778
(87) International publication number: WO 2025/044238

(57) **Abstract**

This application discloses a non-contact sensing method and apparatus, to improve target sensing performance. The method includes: obtaining first information, where the first information is associated with first channel state information CSI and second CSI of a first frequency, the first CSI is CSI of a link from a first node to a second node, and the second CSI is CSI of a link from the second node and the first node; and determining a sensing feature of a target based on the first information.

## Description

### CROSS-REFERENCE TO RELATED APPLICATIONS

This application claims priority to Chinese Patent Application No. 202311112774.9, filed with the China National Intellectual Property Administration on August 30, 2023 and entitled "NON-CONTACT SENSING METHOD, APPARATUS, AND SYSTEM", which is incorporated herein by reference in its entirety.

### TECHNICAL FIELD

This application relates to the field of communication technologies, and in particular, to a non-contact sensing method and apparatus.

### BACKGROUND

With continuous development of communication technologies, communication technologies are increasingly widely applied. Some wireless communication technologies are used to implement non-contact sensing, and the non-contact sensing is widely applied in fields such as industrial production, intelligent transportation, and medical diagnosis. The non-contact sensing means that a feature (for example, a biometric feature or a human body posture) of a target is obtained when the target does not carry any apparatus and is not in contact with an apparatus. In comparison with contact sensing, the non-contact sensing has advantages such as non-intrusiveness, convenience, and low costs.

For example, in some technical solutions, breathing frequency of a user is determined based on received signal strength (Received Signal Strength, RSS) of Wi-Fi. However, small chest motion cannot be sensed based on the RSS, resulting in low detection accuracy of the breathing frequency. In addition, a phase offset phenomenon of commercial Wi-Fi is also a disadvantage of using Wi-Fi to perform breathing detection.

It can be learned that, in a specific application scenario, an existing non-contact sensing method may have problems such as condition mismatch, insufficient accuracy, and poor adaptability.

### SUMMARY

Embodiments of this application provide a non-contact sensing method and apparatus, to improve target sensing performance.

According to a first aspect, an embodiment of this application provides a non-contact sensing method. The method may be executed by a non-contact sensing apparatus. The non-contact sensing apparatus may be an independent device, or may be a chip or a component in a device, or may be software. The non-contact sensing apparatus may be configured in a terminal device. For example, the non-contact sensing apparatus may be an application, and may be installed or run on a chip or a component of the terminal device, or on an intelligent device like a mobile phone or a tablet computer. Alternatively, the non-contact sensing apparatus may be a software module, and may be deployed on a terminal device. Alternatively, the non-contact sensing apparatus may be a newly added hardware module in the terminal device, and related determining logic or an algorithm may be configured in the hardware module, to implement the non-contact sensing method in embodiments of this application.

The method includes: obtaining first information, where the first information is associated with first channel state information CSI and second CSI of a first frequency, the first CSI is CSI of a link from a first node to a second node, and the second CSI is CSI of a link from the second node and the first node; and determining a sensing feature of a target based on the first information.

In a case, the first CSI is the CSI of the link from the first node to the second node, and the second CSI is the CSI of the link from the second node to the first node. It may be understood that the first CSI and the second CSI are CSI in different directions of a same communication link. In other words, the first CSI and the second CSI are CSI in different directions of a same antenna. Correspondingly, in this implementation, the first node and/or the second node may be a single-antenna device. In addition, there is a non-ideal factor or another factor (for example, multipath effect, a frequency offset, or noise interference) on a transmit/receive channel between the first node and the second node, and CSI in different directions between the first node and the second node is different. Therefore, third CSI is subsequently constructed based on the first CSI and the second CSI, so that impact of a phase offset and/or an amplitude offset of unidirectional CSI (namely, the first CSI and/or the second CSI) on a target sensing process can be eliminated, thereby effectively improving target sensing performance.

In another case, the first CSI is CSI on a first communication link from the first node to the second node, and the second CSI is CSI on a second communication link from the first node to the second node. The first communication link is a communication link corresponding to a first antenna, the second communication link is a communication link corresponding to a second antenna, and the first antenna and the second antenna are different antennas between the first node and the second node. Correspondingly, in this implementation, the first node and the second node may be multi-antenna devices. CSI on different communication links is different. Therefore, third CSI is subsequently constructed based on the first CSI and the second CSI, so that impact of a phase offset and/or an amplitude offset of unidirectional CSI (namely, the first CSI and/or the second CSI) on a target sensing process can be eliminated, thereby effectively improving target sensing performance.

It can be learned from the foregoing that the non-contact sensing method provided in embodiments of this application is applicable to the single-antenna device and the multi-antenna device.

In a possible design, a sensing feature of the target includes a physiological indicator and a human body posture. The physiological indicator may include, for example, at least one of an electrocardiogram, a heart rate, respiratory frequency, non-invasive blood pressure, a blood oxygen concentration, or a pulse rate. The human body posture may include, for example, related behaviors such as a fall posture, walking, stillness, a kick action, and a gesture action.

In embodiments of this application, that the first information is associated with the first channel state information CSI and the second CSI of the first frequency may include but is not limited to the following implementations.

Implementation 1: The first information is the first CSI and the second CSI that correspond to the first frequency. Correspondingly, the non-contact sensing apparatus obtains the first CSI and the second CSI in the following cases.

Case 1: When the non-contact sensing method is applied to the first node, the first node receives the first CSI from the second node, where the first CSI is associated with a first sensing signal sent by the first node; and the first node receives a second sensing signal from the second node, and determines the second CSI based on the second sensing signal.

Case 2: When the non-contact sensing method is applied to the second node, the second node receives a first sensing signal from the first node, and determines the first CSI based on the first sensing signal; and the second node receives the second CSI from the first node, where the second CSI is associated with a second sensing signal sent by the second node.

Case 3: When the non-contact sensing method is applied to a third node, the third node receives the first CSI from the second node, where the first CSI is associated with a first sensing signal sent by the first node; and the third node receives the second CSI from the first node, where the second CSI is associated with a second sensing signal sent by the second node.

In Implementation 1, the sensing feature of the target is directly determined based on the first CSI and the second CSI in different directions of the same communication link, so that target sensing performance can be effectively improved.

In Implementation 2, the first information is third CSI or a temporary sensing feature of the target, and the third CSI or the temporary sensing feature of the target is associated with the first CSI and the second CSI. That "the third CSI is associated with the first CSI and the second CSI" may be understood as that the third CSI is determined based on the first CSI and the second CSI, and that "the temporary sensing feature of the target is associated with the first CSI and the second CSI" may be understood as that the temporary sensing feature of the target is determined based on the first CSI and the second CSI. Correspondingly, when the non-contact sensing method is applied to a fourth node, the fourth node may receive the third CSI from the second node or the first node. Alternatively, the fourth node may receive the third CSI from the second node or the first node and receive the temporary sensing feature of the target from the second node or the first node.

In Implementation 2, the fourth node may determine the sensing feature of the target based on the received third CSI, so that target sensing performance can be effectively improved. Alternatively, the fourth node may determine the sensing feature of the target based on the received temporary sensing feature of the target, so that the determined sensing feature of the target is accurate.

In a possible design, the first CSI and the second CSI correspond to a same antenna port pair. That "the first CSI and the second CSI correspond to a same antenna port pair" may be understood as that the first CSI and the second CSI are obtained based on sensing signals transmitted between same transceiver antennas. For example, the first node includes an antenna 1, and the antenna 1 has a transceiver function; and the second node includes an antenna 2, and the antenna 2 has a transceiver function. The first node may send a sensing signal 1 to the second node through the antenna 1, the second node receives the sensing signal 1 through the antenna 2, and the second node may obtain the first CSI through calculation based on the sensing signal 1. In addition, the second node may send a sensing signal 2 to the first node through the antenna 2, the first node receives the sensing signal 2 through the antenna 1, and the first node may obtain the second CSI through calculation based on the sensing signal 2. In short, the first CSI is obtained through calculation based on the sensing signal 1 transmitted between the antenna 1 and the antenna 2, and the second CSI is obtained through calculation based on the sensing signal 2 transmitted between the antenna 2 and the antenna 1.

In a possible design, determining the sensing feature of the target based on the first information includes: constructing third CSI based on the first CSI and the second CSI; and determining the sensing feature based on the third CSI. In this design, impact of phase offsets and amplitude noise of the first CSI and the second CSI on the target sensing process can be eliminated, thereby effectively improving target sensing performance.

In a possible design, constructing the third CSI based on the first CSI and the second CSI includes: performing a first operation on the first CSI and the second CSI, to obtain the third CSI, where the first operation is any one of the following: performing multiplication on the first CSI and the second CSI, performing conjugate multiplication on the first CSI and the second CSI, performing division on the first CSI and the second CSI, or performing conjugate division on the first CSI and the second CSI. In this design, a plurality of manners of eliminating the phase offsets and the amplitude noise of the first CSI and the second CSI are provided, so that the non-contact sensing method provided in embodiments of this application can be flexibly implemented.

In a possible design, determining the sensing feature based on the third CSI includes: extracting an amplitude and/or a phase corresponding to the third CSI; determining a statistical feature of the amplitude and/or a statistical feature of the phase, where the statistical feature of the amplitude includes at least one of a change period, a power spectral density variance, Doppler frequency shift (doppler frequency shift, DFS) information, or the like of the amplitude within first duration, and the statistical feature of the phase includes at least one of a change period, a power spectral density variance, DFS information, or the like of the phase within the first duration; and determining the sensing feature based on the statistical feature of the amplitude and/or the statistical feature of the phase. In this design, the sensing feature of the target may be determined based on the statistical feature of the amplitude and/or the statistical feature of the phase that correspond/corresponds to the third CSI.

In a possible design, determining the sensing feature based on the statistical feature of the amplitude and/or the statistical feature of the phase includes: determining the sensing feature based on the statistical feature of the amplitude when the power spectral density variance of the amplitude is greater than the power spectral density variance of the phase; or determining the sensing feature based on the statistical feature of the phase when the power spectral density variance of the amplitude is less than the power spectral density variance of the phase. That "the power spectral density variance of the amplitude is greater than the power spectral density variance of the phase" may be understood as that power spectral density of the amplitude changes greatly relative to power spectral density of the phase, and "the power spectral density variance of the amplitude is less than the power spectral density variance of the phase" may be understood that the power spectral density of the amplitude changes slightly relative to the power spectral density of the phase. Therefore, in this design, the sensing feature of the target is determined based on a feature that a power spectral density variance of the third CSI changes greatly, so that target sensing performance can be further improved.

In a possible design, when the sensing feature is breathing frequency, determining the sensing feature based on the statistical feature of the amplitude and/or the statistical feature of the phase includes: converting the change period of the amplitude within the first duration and/or the change period of the phase within the first duration into a first waveform; calculating an average time interval between k wave peaks or k wave troughs in the first waveform, where the average time interval represents a time interval between two times of breathing of a user; and determining the breathing frequency based on the average time interval. In this design, the breathing frequency of the target is detected based on the change period of the amplitude within the first duration and/or the change period of the phase within the first duration, so that the determined breathing frequency is accurate.

In a possible design, when the sensing feature is a human body posture, determining the sensing feature based on the statistical feature of the amplitude and/or the statistical feature of the phase includes: inputting the DFS information of the amplitude within the first duration and/or the DFS information of the phase within the first duration into a classifier, to obtain the human body posture. In this design, the human body posture of the target is detected based on the DFS information of the amplitude within the first duration and/or the DFS information of the phase within the first duration, so that the determined human body posture is accurate.

In a possible design, the first CSI is N pieces of CSI, the second CSI is N pieces of CSI, and the third CSI includes N pieces of CSI.

In a possible design, the power spectral density variance of the amplitude of the third CSI corresponding to the first frequency is greater than a first threshold, and/or the power spectral density variance of the phase of the third CSI corresponding to the first frequency is greater than a second threshold.

In a possible design, the first frequency is one of M frequencies, and the method further includes: obtaining first CSI and second CSI that correspond to a frequency other than the first frequency in the M frequencies, where the power spectral density variance of the amplitude of the third CSI corresponding to the first frequency is greater than a power spectral density variance of an amplitude of third CSI corresponding to the another frequency, and the power spectral density variance of the phase of the third CSI corresponding to the first frequency is greater than a power spectral density variance of a phase of the third CSI corresponding to the another frequency. In this design, a frequency with a large power spectral density variance of an amplitude and a large power spectral density variance of a phase is used as the first frequency used to determine the sensing feature of the target, so that target sensing performance can be further improved.

According to a second aspect, an embodiment of this application provides a non-contact sensing apparatus. The apparatus may be an integrated circuit or a chip system. The apparatus has a function of implementing the first aspect or the possible implementations of the first aspect. The function may be implemented by using hardware, or may be implemented by using hardware executing corresponding software. The hardware or the software includes one or more modules/units corresponding to the foregoing functions.

According to a third aspect, an embodiment of this application provides a communication apparatus, including at least one processor and an interface circuit. The interface circuit is configured to: receive a signal from a communication apparatus other than the communication apparatus and transmit the signal to the processor, or send a signal from the processor to a communication apparatus other than the communication apparatus. The processor is configured to implement the method according to the first aspect or the possible designs of the first aspect by using a logic circuit or by executing code instructions. Optionally, the communication apparatus further includes a memory. Alternatively, the memory may be located outside the apparatus. The memory is configured to store a program or code instructions for execution by the at least one processor.

According to a fourth aspect, an embodiment of this application further provides a computer-readable storage medium, including instructions. When the instructions are run on a computer, the computer is enabled to perform the method according to the first aspect or the possible designs of the first aspect.

According to a fifth aspect, an embodiment of this application further provides a computer program product. When the computer program product runs on a computer, the computer is enabled to perform the method according to the first aspect or the possible designs of the first aspect.

According to a sixth aspect, an embodiment of this application further provides a chip system, including a processor. The processor is coupled to a memory, the memory is configured to store a program or instructions, and when the program or the instructions are executed by the processor, the chip system implements the method according to the first aspect or the possible designs of the first aspect. The memory may be located in the chip system, or may be located outside the chip system. In addition, there are one or more processors.

According to a seventh aspect, an embodiment of this application further provides a communication system, including the first node, the second node, the third node, and the fourth node that are configured to perform the method according to the first aspect or the possible designs of the first aspect.

According to an eighth aspect, an embodiment of this application further provides a terminal. The terminal may include the communication apparatus in the second aspect or the third aspect. For example, the terminal includes but is not limited to any one of the following devices: a smart home device (for example, a television, a robotic vacuum cleaner, a smart desk lamp, a speaker system, an intelligent light system, an electrical appliance control system, home background music, a home theater system, an intercom system, or video surveillance), an intelligent transportation device (for example, a vehicle, a ship, an uncrewed aerial vehicle, a train, a van, or a truck), an intelligent manufacturing device (for example, a robot, an industrial device, intelligent logistics, or a smart factory), and a smart terminal (a mobile phone, a computer, a tablet computer, a palmtop computer, a desktop computer, a headset, a speaker, a wearable device, a vehicle-mounted device, a virtual reality device, an augmented reality device, or the like).

It should be understood that, for technical effect of the second aspect to the eighth aspect, refer to corresponding technical effect descriptions in the first aspect. Details are not described herein again.

### BRIEF DESCRIPTION OF DRAWINGS

FIG. 1 is a diagram of a possible system architecture according to an embodiment of this application;
FIG. 2 is a schematic flowchart of a non-contact sensing method according to an embodiment of this application;
FIG. 3 is a schematic flowchart of another non-contact sensing method according to an embodiment of this application;
FIG. 4 is a schematic flowchart of another non-contact sensing method according to an embodiment of this application;
FIG. 5 is a schematic flowchart of another non-contact sensing method according to an embodiment of this application;
FIG. 6 is a schematic flowchart of another non-contact sensing method according to an embodiment of this application;
FIG. 7A is a diagram of a possible scenario according to an embodiment of this application;
FIG. 7B is a diagram of signal sending according to an embodiment of this application;
FIG. 8 is a schematic flowchart of another non-contact sensing method according to an embodiment of this application;
FIG. 9A is a diagram 1 of a waveform according to an embodiment of this application;
FIG. 9B is a diagram 2 of a waveform according to an embodiment of this application;
FIG. 9C is a diagram 3 of a waveform according to an embodiment of this application;
FIG. 10 is a diagram of another possible scenario according to an embodiment of this application;
FIG. 11 is a schematic flowchart of another non-contact sensing method according to an embodiment of this application;
FIG. 12 is a schematic flowchart of human body posture recognition according to an embodiment of this application;
FIG. 13 is a diagram of a structure of a non-contact sensing apparatus according to an embodiment of this application; and
FIG. 14 is a diagram of a structure of a chip system according to an embodiment of this application.

### DESCRIPTION OF EMBODIMENTS

To make the objectives, technical solutions, and advantages of embodiments of this application clearer, the following further describes embodiments of this application in detail with reference to the accompanying drawings.

The following describes some terms in embodiments of this application, to facilitate understanding of a person skilled in the art.
(1) Channel state information (Channel State Information, CSI) is used to describe a characteristic and a status of a wireless channel. In wireless communication, a signal is affected, in a propagation process, by a plurality of factors such as multipath propagation, fading, interference, and a non-ideal factor of a transceiver apparatus of a transceiver. These factors cause a characteristic of a channel (or an equivalent channel) to change with time and space. The CSI provides information about a channel, including an amplitude, a phase, a frequency response, and the like. In some embodiments, the CSI includes a channel gain matrix H, and the channel gain matrix H represents a channel characteristic when a signal is transmitted from a transmit antenna to a receive antenna. In a MIMO (multiple-input multiple-output) system with M transmit antennas and N receive antennas, H is an N×M matrix, where each element represents a channel gain or a channel coefficient from the transmit antenna to the receive antenna.
(2) A target is a to-be-sensed person, object, animal, or the like. For example, in a health monitoring scenario, the target may be a person. For another example, in a tailgate kicking detection scenario of a vehicle, the target may be a person and/or a trunk of the vehicle.
(3) A sensing feature of the target is a feature of the target that needs to be sensed. In embodiments of this application, the sensing feature of the target includes a physiological indicator and a human body posture. The physiological indicator may include, for example, at least one of a heart rate, an electrocardiogram, respiratory frequency, non-invasive blood pressure, a blood oxygen concentration, a pulse rate, or the like. The human body posture may include, for example, related behaviors such as a fall posture, walking, stillness, a kick action, and a gesture action.
(4) "At least one" means one or more, and "a plurality of" means two or more. "And/or" describes an association relationship between associated objects and indicates that three relationships may exist. For example, A and/or B may indicate the following three cases: Only A exists, both A and B exist, and only B exists, where A and B may be singular or plural. The character "/" generally indicates an "or" relationship between the associated objects. "At least one of the following items (pieces)" or a similar expression thereof indicates any combination of these items, including a single item (piece) or any combination of a plurality of items (pieces). For example, at least one item (piece) of a, b, or c may indicate: a, b, c, a and b, a and c, b and c, or a, b, and c, where a, b, and c may be singular or plural. In addition, unless otherwise stated, ordinal numbers such as "first" and "second" in embodiments of this application are for distinguishing between a plurality of objects, but are not intended to limit an order, a time sequence, priorities, or importance of the plurality of objects. For example, first CSI and second CSI are merely used to distinguish between different CSI, but do not indicate that the two types of CSI are different in content, priorities, sending sequences, importance, or the like.

The foregoing describes some concepts in embodiments of this application, and the following describes technical features in embodiments of this application.

Embodiments of this application provide a non-contact sensing method, apparatus, and system, to improve target sensing performance. In the method, a sensing feature of a target is determined based on first information associated with a plurality of pieces of CSI (namely, first CSI and second CSI), so that impact of a phase shift and/or an amplitude shift of unidirectional CSI on a target sensing process can be eliminated. In this way, target sensing performance is effectively improved. In addition, the first CSI and the second CSI may be CSI in different directions of a same communication link, so that the non-contact sensing method provided in this embodiment of this application may be applicable to a single-antenna receive end device and a multi-antenna receive end device, thereby effectively improving an application scope of non-contact sensing. In the following, the method and the apparatus are based on a same technical concept. Because a problem-resolving principle of the method is similar to that of the device, mutual reference may be made to implementations of the device and the method, and repeated parts are not described again.

The non-contact sensing method provided in embodiments of this application may be applicable to any scenario in which target sensing is required. For example, the scenario may be a scenario like an in-vehicle wireless sensing scenario, an indoor health monitoring scenario, an intrusion detection scenario, a remote medical diagnosis scenario, a breathing frequency detection scenario, or a gesture control game scenario.

In addition, the non-contact sensing method provided in embodiments of this application is applicable to a communication system based on an orthogonal frequency division multiplexing (orthogonal frequency division multiplexing, OFDM) waveform, for example, a wireless fidelity (wireless fidelity, WI-FI) system, a new radio (New Radio, NR) access technology system, a long term evolution (long term evolution, LTE) system, or a SparkLink basic (sparklink basic, SLB) system. The method is also applicable to a frequency hopping system, like a Bluetooth (Bluetooth) system and a SparkLink low energy (sparklink low energy, SLE) system.

The following describes a system architecture applicable to embodiments of this application.

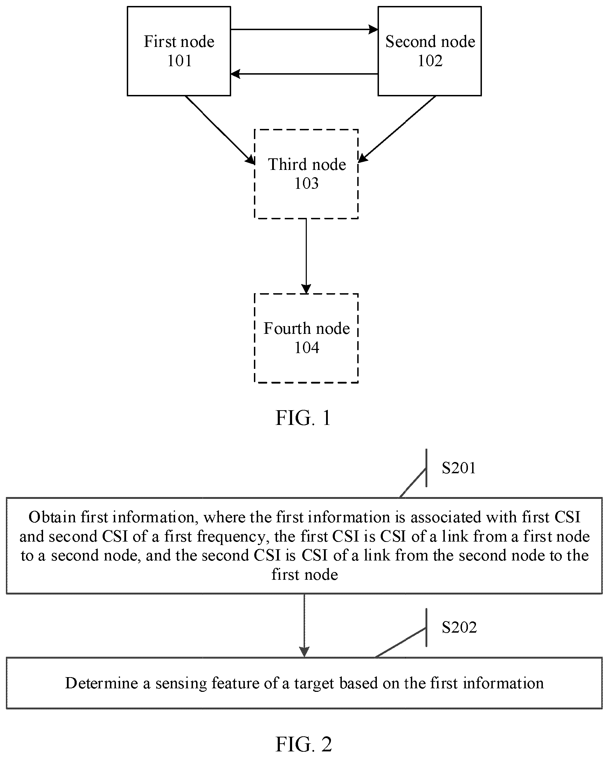

FIG. 1 is a diagram of an architecture of a communication system to which an embodiment of this application is applicable.

Refer to FIG. 1. The system includes a first node 101 and a second node 102, where information exchange may be performed between the second node 102 and the first node 101, so that the second node 102 or the first node 101 may determine a sensing feature of a target. In an optional implementation, the system may further include a third node 103, where information exchange may be performed between the first node 101, the second node 102, and the third node 103, so that the third node 103 may determine the sensing feature of the target. In another optional implementation, the system may further include a fourth node 104, where information exchange may be performed between the first node 101, the second node 102, the third node 103, and the fourth node 104, so that the fourth node 104 may determine the sensing feature of the target.

The first node 101, the second node 102, the third node 103, and the fourth node 104 may be understood as a module or an apparatus having a corresponding function, or the first node 101, the second node 102, the third node 103, and the fourth node 104 may be any communication node. In a design, the communication node may be a short-range communication node, for example, any one of a Bluetooth terminal node, a SparkLink short-range communication terminal node, or a wireless fidelity (wireless fidelity, Wi-Fi) terminal node. This is not specifically limited in embodiments of this application. Any one of the first node 101, the second node 102, the third node 103, and the fourth node 104 may be a T node or a G node in short-range communication.

It may be understood that the architecture shown in FIG. 1 may be applied to a plurality of communication scenarios, for example, a 5th generation (5th Generation, 5G) communication system, a future 6th generation communication system and another evolved communication system, a long term evolution (long term evolution, LTE) communication system, vehicle to everything (vehicle to everything, V2X), long term evolution-vehicle (LTE-vehicle, LTE-V), vehicle to vehicle (vehicle to vehicle, V2V), internet of vehicles, machine type communication (machine type communication, MTC), internet of things (internet of things, IoT), long term evolution-machine to machine (LTE-machine to machine, LTE-M), and machine to machine (machine to machine, M2M). This is not limited in this application.

Based on the communication system shown in FIG. 1, an embodiment of this application provides a non-contact sensing method. The method may be executed by a non-contact sensing apparatus. The non-contact sensing apparatus may be an independent device, or may be a chip or a component in a device, or may be software. The non-contact sensing apparatus may be configured in a terminal device. For example, the non-contact sensing apparatus may be an application, and may be installed or run on a chip or a component of the terminal device, or on an intelligent device like a mobile phone or a tablet computer in the terminal device. Alternatively, the non-contact sensing apparatus may be a software module, and may be deployed on a terminal device. Alternatively, the non-contact sensing apparatus may be a newly added hardware module in the terminal device, and related determining logic or an algorithm may be configured in the hardware module, to implement the non-contact sensing method in embodiments of this application.

FIG. 2 is a schematic flowchart of a non-contact sensing method according to an embodiment of this application. The method may be executed by a non-contact sensing apparatus. When the non-contact sensing apparatus may be deployed on any one of the first node 101, the second node 102, the third node 103, or the fourth node 104, the method may be executed by any one of the first node 101, the second node 102, the third node 103, or the fourth node 104 in FIG. 1. The method includes the following steps.

S201: Obtain first information, where the first information is associated with first CSI and second CSI of a first frequency, the first CSI is CSI of a link from a first node to a second node, and the second CSI is CSI of a link from the second node and the first node.

In a possible implementation, the first CSI is the CSI of the link from the first node to the second node, and the second CSI is the CSI of the link from the second node to the first node. It may be understood that the first CSI and the second CSI are CSI in different directions of a same communication link. In other words, the first CSI and the second CSI are CSI in different directions of a same antenna. Correspondingly, in this implementation, the first node and/or the second node may be a single-antenna device. In addition, there is a non-ideal factor or another factor (for example, multipath effect, a frequency offset, or noise interference) on a transmit/receive channel between the first node and the second node, and CSI in different directions between the first node and the second node is different. Therefore, third CSI is subsequently constructed based on the first CSI and the second CSI, so that impact of a phase offset and/or an amplitude offset of unidirectional CSI (namely, the first CSI and/or the second CSI) on a target sensing process can be eliminated, thereby effectively improving target sensing performance.

In another possible implementation, the first CSI is CSI on a first communication link from the first node to the second node, and the second CSI is CSI on a second communication link from the first node to the second node. The first communication link is a communication link corresponding to a first antenna, the second communication link is a communication link corresponding to a second antenna, and the first antenna and the second antenna are different antennas between the first node and the second node. Correspondingly, in this implementation, the first node and the second node may be multi-antenna devices. CSI on different communication links is different. Therefore, third CSI is subsequently constructed based on the first CSI and the second CSI, so that impact of a phase offset and/or an amplitude offset of unidirectional CSI (namely, the first CSI and/or the second CSI) on a target sensing process can be eliminated, thereby effectively improving target sensing performance.

It can be learned from the foregoing that the non-contact sensing method provided in embodiments of this application is applicable to the single-antenna device and the multi-antenna device.

In this embodiment of this application, the first frequency may be one or more frequencies. For example, when the first frequency is G frequencies, first CSI and second CSI that correspond to each of the G frequencies may be determined. One piece of third CSI is constructed based on the first CSI and the second CSI that correspond to each frequency, and correspondingly, G pieces of third CSI may be obtained. Further, one temporary sensing feature of the target may be obtained based on each piece of third CSI, and then a final sensing feature of the target may be determined from G temporary sensing features that are of the target and that correspond to the G pieces of third CSI. G a positive integer greater than or equal to 2.

Optionally, that "the first CSI and the second CSI correspond to a same antenna port pair" may be understood as that the first CSI and the second CSI are obtained based on sensing signals transmitted between same transceiver antennas. For example, the first node 101 includes an antenna 1, and the antenna 1 has a transceiver function; and the second node 102 includes an antenna 2, and the antenna 2 has a transceiver function. The first node 101 may send a sensing signal 1 to the second node 102 through the antenna 1, the second node 102 receives the sensing signal 1 through the antenna 2, and the second node 102 may obtain the first CSI through calculation based on the sensing signal 1. In addition, the second node 102 may send a sensing signal 2 to the first node 101 through the antenna 2, the first node 101 receives the sensing signal 2 through the antenna 1, and the first node 101 may obtain the second CSI through calculation based on the sensing signal 2. In short, the first CSI is obtained through calculation based on the sensing signal 1 transmitted between the antenna 1 and the antenna 2, and the second CSI is obtained through calculation based on the sensing signal 2 transmitted between the antenna 2 and the antenna 1.

S202: Determine a sensing feature of a target based on the first information.

In S202, the sensing feature of the target is determined based on the first information associated with a plurality of pieces of CSI (namely, the first CSI and the second CSI), so that impact of a phase shift and/or an amplitude shift of unidirectional CSI (namely, the first CSI and/or the second CSI) on a target sensing process can be eliminated. In this way, target sensing performance is effectively improved.

In embodiments of this application that the first information is associated with the first channel state information CSI and the second CSI of the first frequency may include but is not limited to the following cases.

Case 1: The first information is the first CSI and the second CSI that correspond to the first frequency.

Case 2: The first information is the third CSI or the temporary sensing feature of the target, and the third CSI or the temporary sensing feature of the target is associated with the first CSI and the second CSI. That "the third CSI is associated with the first CSI and the second CSI" may be understood as that the third CSI is determined based on the first CSI and the second CSI, and that "the temporary sensing feature of the target is associated with the first CSI and the second CSI" may be understood as that the temporary sensing feature of the target is determined based on the first CSI and the second CSI.

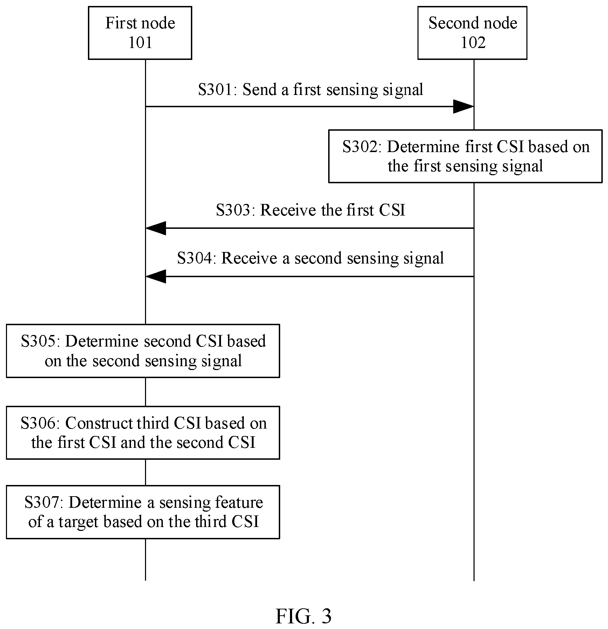

With reference to a specific case of the first information, a specific implementation procedure of the non-contact sensing method in embodiments of this application may include different cases shown in the following a method example 1 to a method example 4. The following separately provides descriptions with reference to FIG. 3 to FIG. 6. It may be understood that the steps in FIG. 3 to FIG. 6 are merely examples for describing steps that may be included in the non-contact sensing method in embodiments of this application, but do not constitute any limitation. Specific execution sequences of the steps in the examples may be adjusted mutually. In some embodiments, technical features in different embodiments described with reference to FIG. 3 to FIG. 6 may further be combined into a new embodiment.

Method example 1: The first information is the first CSI and the second CSI that correspond to the first frequency, and the first node 101 is used as a node for determining the sensing feature of the target. In this case, the non-contact sensing apparatus is deployed on the first node 101.

As shown in FIG. 3, in the method example 1, the non-contact sensing method provided in this embodiment of this application may include the following steps.

S301: A first node 101 sends a first sensing signal to a second node 102. Correspondingly, the second node 102 receives the first sensing signal.

S302: The second node 102 determines first CSI based on the first sensing signal.

The first sensing signal received by the second node 102 is a signal that passes through an air interface channel, and in this case, the first sensing signal includes information used to estimate a channel status.

During specific implementation of S302, the second node 102 may analyze the received first sensing signal to extract information about signal distortion, multipath effect, interference, and noise, and estimate a channel characteristic based on the information; and then the second node 102 extracts the first CSI from the estimated channel characteristic. The first CSI may include at least one of a channel gain, a phase, an amplitude, a frequency response, or another parameter that describes a channel behavior.

S303: The second node 102 sends the first CSI to the first node 101. Correspondingly, the first node 101 receives the first CSI.

In S303, the second node 102 feeds back the first CSI to the first node 101. In one aspect, the first node 101 may perform a subsequent target sensing operation based on the first CSI. In another aspect, the first node 101 may adjust a sending parameter based on the first CSI, for example, adjust transmit power, select a proper coding and modulation scheme, or apply a beamforming technology.

S304: The second node 102 sends a second sensing signal to the first node 101. Correspondingly, the first node 101 receives the second sensing signal.

The first sensing signal and the second sensing signal are pilot signals known to the first node 101 and the second node 102 or other signals known to the first node 101 and the second node 102. In other words, the first sensing signal and the second sensing signal are signals known to a receive end and a transmit end.

S305: The first node 101 determines second CSI based on the second sensing signal.

The second sensing signal received by the first node 101 is a signal that passes through an air interface channel, and in this case, the second sensing signal includes information used to estimate a channel status.

During specific implementation of S305, the first node 101 may analyze the received second sensing signal to extract information about signal distortion, multipath effect, interference, and noise, and estimate a channel characteristic based on the information; and then the first node 101 extracts the second CSI from the estimated channel characteristic. The second CSI may include any one of a channel gain, a phase, an amplitude, a frequency response, or another parameter that describes a channel behavior.

S306: The first node 101 constructs third CSI based on the first CSI and the second CSI.

In S306, the first node 101 constructs the third CSI based on the first CSI and the second CSI, so that impact of a phase offset and/or an amplitude offset of the unidirectional CSI (namely, the first CSI and/or the second CSI) on the target sensing process can be eliminated or reduced, thereby effectively improving target sensing performance.

During specific implementation of S306, the first node 101 may perform a first operation on the first CSI and the second CSI to obtain the third CSI. The first operation is any one of the following: performing multiplication on the first CSI and the second CSI, performing conjugate multiplication on the first CSI and the second CSI, performing division on the first CSI and the second CSI, performing conjugate division on the first CSI and the second CSI, or calculating a ratio between the first CSI and the second CSI.

For example, the first CSI is h_{T}=h×e^{i_{φ}G}×e^{-j_{φ}T}, the second CSI is h_{G}=h×e^{j_{φT}}e^{-j_{φ}G}, and the third CSI may be any one of h_{G}×h_{T}, sqrt(h_{G}×h_{T}), (h_{G×}h_{T}).^(1/2), h_{G}×h*_{T}, sqrt(h_{G}×h*_{T}), (h_{G}×h*_{T}).^(1/2), h_{T}/h_{G}, h_{G}/h_{T}, and the like.

S307: The first node 101 determines the sensing feature of the target based on the third CSI.

During specific implementation of S307, the first node 101 may extract an amplitude and/or a phase corresponding to the third CSI, determine a statistical feature of an amplitude and/or a statistical feature of a phase, and determine the sensing feature based on the statistical feature of the amplitude and/or the statistical feature of the phase.

In embodiments of this application, the first CSI may be N pieces of CSI, the second CSI may be N pieces of CSI, and the third CSI may be N pieces of CSI. Correspondingly, the statistical feature of the amplitude corresponding to the third CSI includes at least one of a change period, a power spectral density variance, or Doppler frequency shift (doppler frequency shift, DFS) information of the amplitude within first duration. The statistical feature of the phase corresponding to the third CSI includes at least one of a change period, a power spectral density variance, or DFS information of the phase within the first duration. In this case, the sensing feature of the target may be determined based on the statistical feature of the amplitude and/or the statistical feature of the phase that correspond/corresponds to the third CSI. The first duration may be 10 minutes, 5 minutes, 1 minute, or the like.

In a possible implementation, the first node 101 may perform smoothing and standardization processing on the amplitude feature and the phase feature by using an S-G filtering method and a Zscore standardization method; and convert, by using fast Fourier transform, the amplitude feature and the phase feature that are time-domain signals into corresponding frequency-domain signals, to obtain power spectral density corresponding to each of the frequency-domain signals, and to obtain the corresponding power spectral density variances.

Further, when the power spectral density variance of the amplitude is greater than the power spectral density variance of the phase, the first node 101 may determine the sensing feature based on the statistical feature of the amplitude; or when the power spectral density variance of the amplitude is less than the power spectral density variance of the phase, the first node 101 may determine the sensing feature based on the statistical feature of the phase. That "the power spectral density variance of the amplitude is greater than the power spectral density variance of the phase" may be understood as that power spectral density of the amplitude changes greatly relative to power spectral density of the phase, and "the power spectral density variance of the amplitude is less than the power spectral density variance of the phase" may be understood that the power spectral density of the amplitude changes slightly relative to the power spectral density of the phase. If the power spectral density variance changes greatly, it means that the feature is sensitive to signal sensing. Therefore, the first node 101 may determine the sensing feature of the target based on a feature in which the power spectral density variance of the third CSI changes greatly, so that target sensing performance can be further improved.

Method example 2: The first information is the first CSI and the second CSI that correspond to the first frequency, and the second node 102 is used as a node for determining the sensing feature of the target. In this case, the non-contact sensing apparatus is deployed on the second node 102.

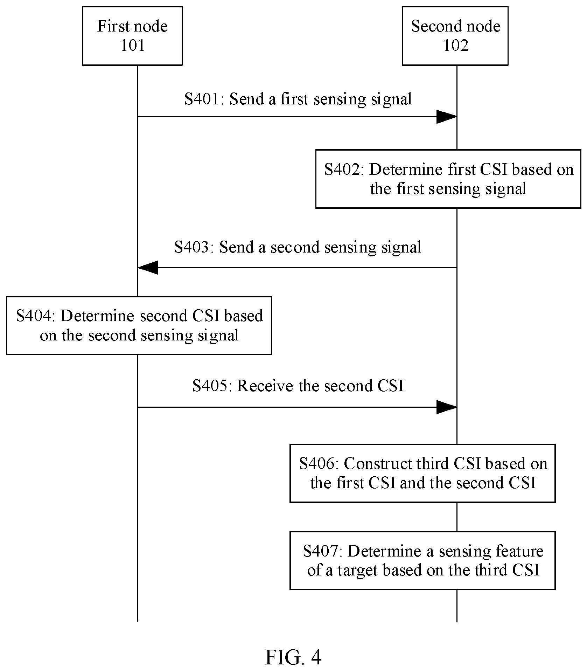

As shown in FIG. 4, in the method example 2, the non-contact sensing method provided in this embodiment of this application may include the following steps.

S401: A first node 101 sends a first sensing signal to a second node 102. Correspondingly, the second node 102 receives the first sensing signal.

S402: The second node 102 determines first CSI based on the first sensing signal.

For a specific implementation of S402, refer to the foregoing related description of S302. Details are not described herein again.

S403: The second node 102 sends a second sensing signal to the first node 101. Correspondingly, the first node 101 receives the second sensing signal.

S404: The first node 101 determines second CSI based on the second sensing signal.

For a specific implementation of S404, refer to the foregoing related description of S305. Details are not described herein again.

S405: The first node 101 sends the second CSI to the second node 102. Correspondingly, the second node 102 receives the second CSI.

In S405, the first node 101 feeds back the second CSI to the second node 102. In one aspect, the second node 102 may perform a subsequent target sensing operation based on the first CSI. In another aspect, the second node 102 may adjust a sending parameter based on the second CSI, for example, adjust transmit power, select a proper coding and modulation scheme, or apply a beamforming technology.

S406: The second node 102 constructs third CSI based on the first CSI and the second CSI.

S407: The second node 102 determines the sensing feature of the target based on the third CSI.

For specific implementations of S406 and S407, refer to related descriptions of the specific implementations of S306 and S307. The first node 101 is replaced with the second node 102. Details are not described herein again.

Method example 3: The first information is the first CSI and the second CSI that correspond to the first frequency, and the third node 103 is used as a node for determining the sensing feature of the target. In this case, the non-contact sensing apparatus is deployed on the third node 103.

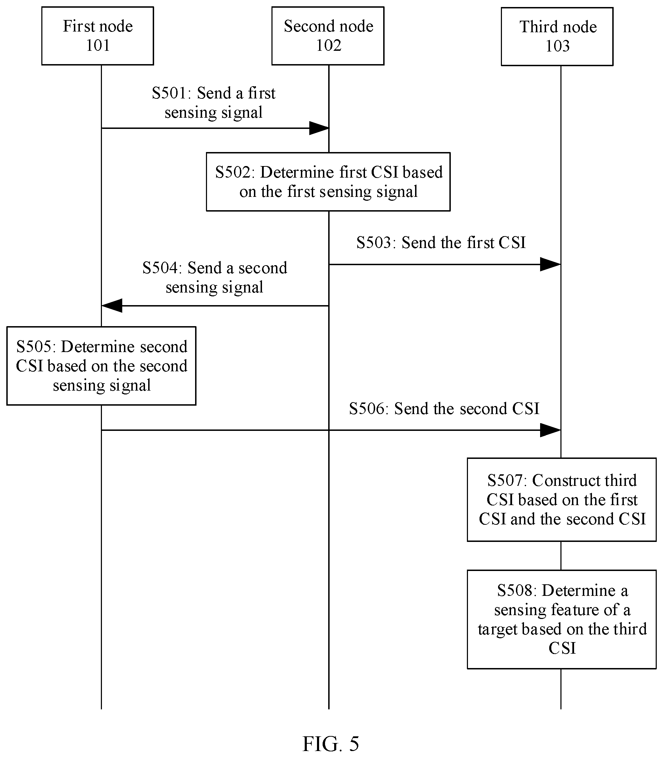

As shown in FIG. 5, in the method example 3, the non-contact sensing method provided in this embodiment of this application may include the following steps.

S501: A first node 101 sends a first sensing signal to a second node 102. Correspondingly, the second node 102 receives the first sensing signal.

S502: The second node 102 determines first CSI based on the first sensing signal.

For a specific implementation of S502, refer to the foregoing related description of S302. Details are not described herein again.

S503: The second node 102 sends the first CSI to a third node 103. Correspondingly, the third node 103 receives the first CSI.

In S503, the second node 102 sends the first CSI to the third node 103, so that the third node 103 may perform a subsequent target sensing procedure based on the first CSI.

S504: The second node 102 sends a second sensing signal to the first node 101. Correspondingly, the first node 101 receives the second sensing signal.

S505: The first node 101 determines second CSI based on the second sensing signal.

For a specific implementation of S505, refer to the foregoing related description of S305. Details are not described herein again.

S506: The first node 101 sends the second CSI to the third node 103. Correspondingly, the third node 103 receives the second CSI.

In S506, the first node 101 sends the second CSI to the third node 103, so that the third node 103 may perform a subsequent target sensing procedure based on the second CSI.

S507: The third node 103 constructs third CSI based on the first CSI and the second CSI.

S508: The third node 103 determines a sensing feature of a target based on the third CSI.

For specific implementations of S507 and S508 refer to related descriptions of the specific implementations of S306 and S307. The first node 101 is replaced with the third node 103. Details are not described herein again.

Method example 4: The first information is third CSI or a temporary sensing feature of the target, and a fourth node 104 is used as a node for determining a sensing feature of the target. In this case, the non-contact sensing apparatus is deployed on the fourth node 104.

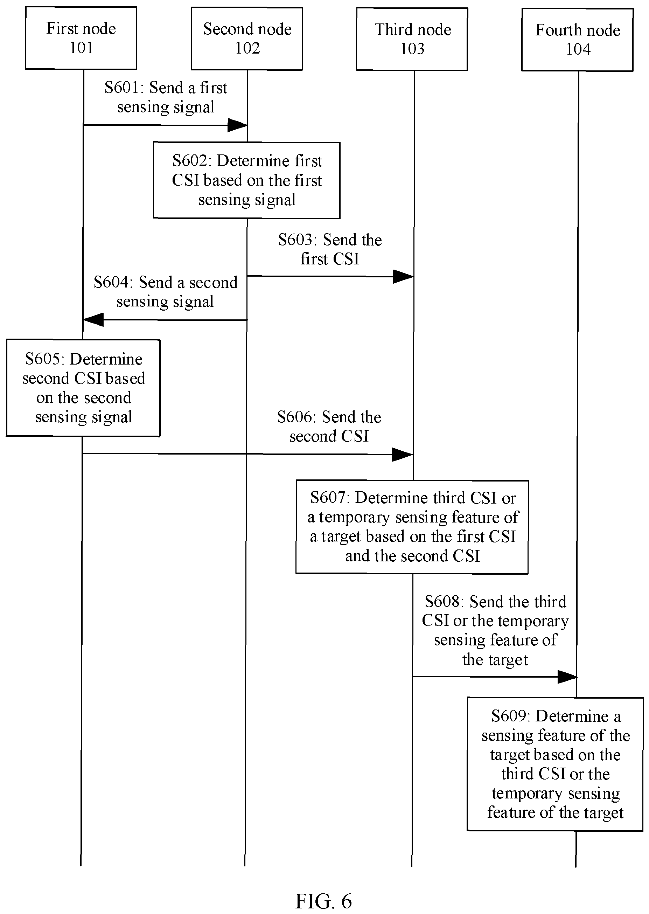

As shown in FIG. 6, in the method example 4, the non-contact sensing method provided in this embodiment of this application may include the following steps.

S601: A first node 101 sends a first sensing signal to a second node 102. Correspondingly, the second node 102 receives the first sensing signal.

S602: The second node 102 determines first CSI based on the first sensing signal.

For a specific implementation of S602, refer to the foregoing related description of S302. Details are not described herein again.

S603: The first node 101 sends the first CSI to a third node 103. Correspondingly, the third node 103 receives the first CSI.

S604: The second node 102 sends a second sensing signal to the first node 101. Correspondingly, the first node 101 receives the second sensing signal.

S605: The first node 101 determines second CSI based on the second sensing signal.

For a specific implementation of S605, refer to the foregoing related description of S305. Details are not described herein again.

S606: The first node 101 sends the second CSI to the third node 103. Correspondingly, the third node 103 receives the second CSI.

S607: The third node 103 determines third CSI or a temporary sensing feature of a target based on the first CSI and the second CSI.

For a specific implementation in which the third node 103 determines the third CSI based on the first CSI and the second CSI, refer to related descriptions of the specific implementation of S306. The first node 101 is replaced with the third node 103. This is not described herein again.

A specific implementation process in which the third node 103 determines the temporary sensing feature of the target based on the first CSI and the second CSI may be: The third node 103 constructs the third CSI based on the first CSI and the second CSI; and determines the temporary sensing feature of the target based on the third CSI.

S608: The third node 103 sends the third CSI or the temporary sensing feature of the target to a fourth node 104. The fourth node 104 receives the third CSI or the temporary sensing feature of the target.

S609: The fourth node 104 determines a sensing feature of the target based on the third CSI or the temporary sensing feature of the target.

For a specific implementation in which the fourth node 104 determines the sensing feature of the target based on the third CSI, refer to related descriptions of the specific implementation of S307. The first node 101 is replaced with the fourth node 104. This is not described herein again.

In a possible implementation, the temporary sensing feature of the target includes one or more temporary sensing features. The fourth node 104 needs to screen out, from the one or more temporary sensing features, a temporary sensing feature whose confidence is greater than a preset threshold, and use the temporary sensing feature whose confidence is greater than the preset threshold as the sensing feature of the target.

In a possible implementation, the fourth node 104 may input the temporary sensing feature of the target into a sensing feature recognition model, and determine the sensing feature of the target based on a recognition result of the sensing feature recognition model. The sensing feature recognition model may be pre-trained. For example, the sensing feature recognition model is a human body posture recognition model, and the temporary sensing feature of the target is a fall posture. The fourth node 104 may input the fall posture into the human body posture recognition model for further recognition. If a confidence output by the human body posture recognition model when the fall posture is recognized is greater than 0.9, the fall posture is used as the sensing feature of the target.

In the foregoing examples 1 to 4, the first CSI and the second CSI that correspond to the first frequency are used as examples to describe a process in which the non-contact sensing apparatus determines the sensing feature of the target.

In some other embodiments of this application, the non-contact sensing apparatus may further obtain first CSI and second CSI of M frequencies, and determine third CSI corresponding to the M frequencies. In this way, the non-contact sensing apparatus may determine, from the M frequencies, the first frequency that meets a first condition. When the first frequency meets the first condition, it may be understood that the first frequency is sensitive to a signal change, so that the sensing feature of the target determined based on the first frequency is accurate.

The first condition may include but is not limited to the following cases.

Case 1: The first condition may be that a power spectral density variance of an amplitude of the third CSI corresponding to the first frequency is greater than a power spectral density variance of an amplitude of third CSI corresponding to another frequency in the M frequencies, and/or a power spectral density variance of a phase of the third CSI corresponding to the first frequency is greater than a power spectral density variance of a phase of third CSI corresponding to another frequency in the M frequencies.

For example, the M frequencies include a frequency 1, a frequency 2, a frequency 3, and a frequency 4, where a power spectral density variance of an amplitude of third CSI corresponding to the frequency 1 is greater than power spectral density variances of amplitudes of third CSI corresponding to the frequency 2, the frequency 3, and the frequency 4, and a power spectral density variance of a phase of the third CSI corresponding to the frequency 1 is greater than power spectral density variances of phases of the third CSI corresponding to the frequency 2, the frequency 3, and the frequency 4. The non-contact sensing apparatus determines the sensing feature of the target based on the third CSI corresponding to the frequency 1.

Case 2: The first condition may be that the power spectral density variance of the amplitude of the third CSI corresponding to the first frequency is greater than a first threshold, and/or the power spectral density variance of the phase of the third CSI corresponding to the first frequency is greater than a second threshold. Specific values of the first threshold and the second threshold may be the same or different.

Example 1: The M frequencies include a frequency 1, a frequency 2, a frequency 3, and a frequency 4, where power spectral density variances of amplitudes of third CSI of the frequency 1 and the frequency 2 are greater than a first threshold, and power spectral density variances of phases of the third CSI corresponding to the frequency 1 and the frequency 2 are greater than a second threshold. In this case, the non-contact sensing apparatus determines the sensing feature of the target based on the third CSI corresponding to the frequency 1 and the frequency 2.

Example 2: The M frequencies include a frequency 1, a frequency 2, and a frequency 3, and power spectral density variances of amplitudes of third CSI of the frequency 1 and the frequency 2 are greater than a first threshold. In this case, the non-contact sensing apparatus determines the sensing feature of the target based on the third CSI corresponding to the frequency 1 and the frequency 2.

Example 3: The M frequencies include a frequency 1, a frequency 2, a frequency 3, and a frequency 4, where power spectral density variances of phases of third CSI corresponding to the frequency 1 and the frequency 2 are greater than a second threshold. In this case, the non-contact sensing apparatus determines the sensing feature of the target based on the third CSI corresponding to the frequency 1 and the frequency 2.

For ease of understanding, the following further describes the non-contact sensing method provided in embodiments of this application with reference to a specific example.

Example 1: An example in which the sensing feature of the target is breathing frequency of a user is used.

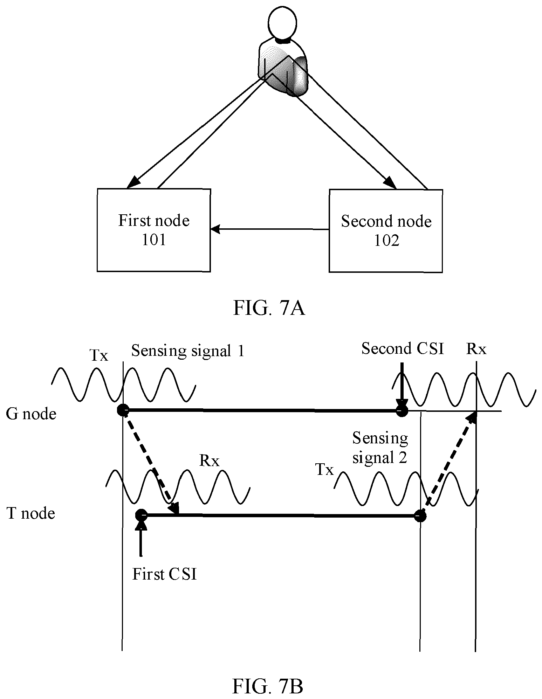

FIG. 7A is a diagram of a scenario in which non-contact sensing is applied to indoor monitoring. The scenario includes a first node 101, a second node 102, and a user. The first node 101, the second node 102, and the user may be located in same indoor space. When the user breathes, chest fluctuation motion affects CSI of a signal transmitted between the first node 101 and the second node 102. Therefore, the first node 101 or the second node 102 may detect breathing frequency of the user based on the CSI of the signal transmitted between the first node 101 and the second node 102. When the first node 101 is a G node and the second node 102 is a T node, a schematic flowchart of signal transmission between the first node 101 and the second node 102 is shown in FIG. 7B. The G node sends a sensing signal 1 to the T node, and the T node determines first CSI based on the sensing signal 1. Then, the T node sends a sensing signal 2 to the G node, and the G node determines second CSI based on the sensing signal 2.

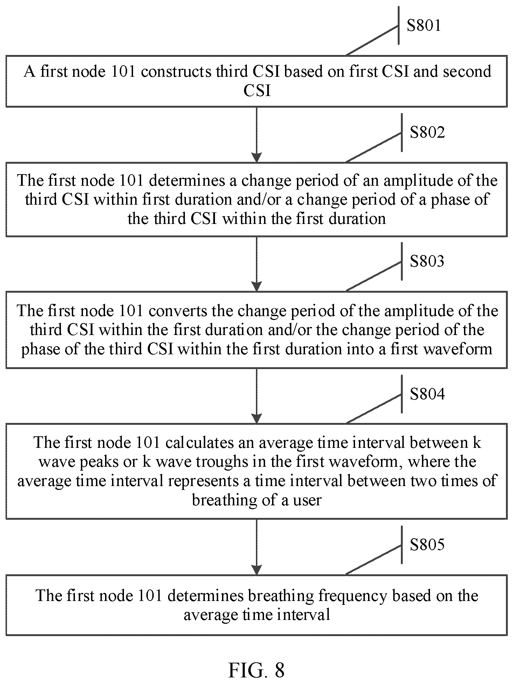

As shown in FIG. 8, that the first node 101 determines the breathing frequency of the user based on the CSI of the signal transmitted between the first node 101 and the second node 102 includes the following procedure.

S801: The first node 101 constructs third CSI based on the first CSI and the second CSI.

In S801, the first node 101 constructs the third CSI based on the first CSI and the second CSI, so that impact of phase offsets and/or amplitude offsets of the first CSI and the second CSI on a breathing detection process can be eliminated, and noise levels in amplitudes of the first CSI and the second CSI can be reduced, thereby greatly extending a sensing range of breathing detection.

S802: The first node 101 determines a change period of an amplitude of the third CSI within first duration and/or a change period of a phase of the third CSI within the first duration.

The first duration may be, for example, 1 minute, 5 minutes, 10 minutes, or the like.

S803: The first node 101 converts the change period of the amplitude of the third CSI within the first duration and/or the change period of the phase of the third CSI within the first duration into a first waveform.

S804: The first node 101 calculates an average time interval between k wave peaks or k wave troughs in the first waveform, where the average time interval represents a time interval between two times of breathing of the user.

S805: The first node 101 determines the breathing frequency based on the average time interval.

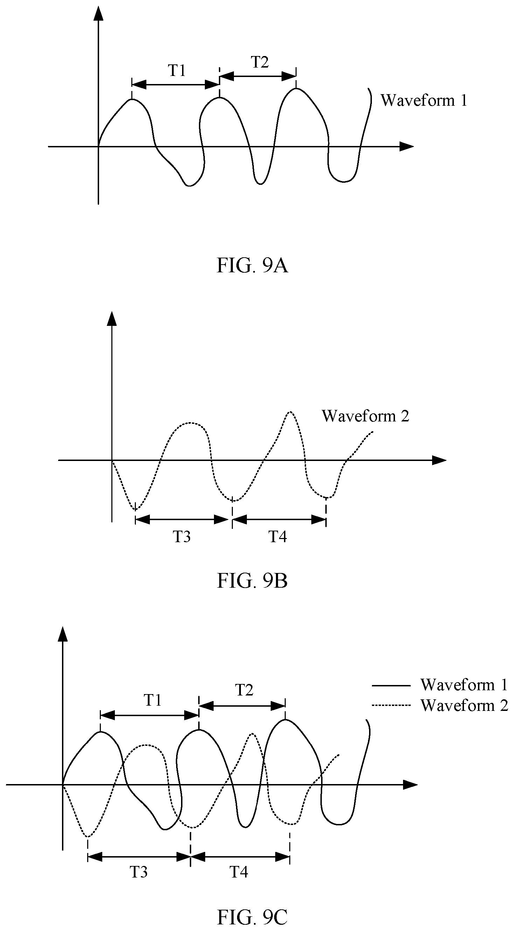

For example, FIG. 9A shows a diagram of a waveform 1 corresponding to a change period of an amplitude of the third CSI within 5 minutes. In FIG. 9A, a time interval between a wave peak 1 and a wave peak 2 of the waveform 1 is T1, and a time interval between a wave peak 2 and a wave peak 3 of the waveform 1 is T2. In this case, the first node 101 may obtain an average time interval (T1+T2)/2, and use (T1+T2)/2 as a time interval between two times of breathing of the user. In this way, the first node 101 may determine the breathing frequency of the user based on the time interval.

For another example, FIG. 9B shows a diagram of a waveform 2 corresponding to a change period of a phase of the third CSI within 5 minutes. In FIG. 9B, a time interval between a wave trough 1 and a wave trough 2 of the waveform 2 is T3, and a time interval between a wave trough 2 and a wave trough 3 of the waveform 2 is T4. In this case, the first node 101 may obtain an average time interval (T3+T4)/2, and use (T3+T4)/2 as a time interval between two times of breathing of the user. In this way, the first node 101 may determine the breathing frequency of the user based on the time interval.

For another example, FIG. 9C shows the diagram of the waveform 1 corresponding to the change period of the amplitude of the third CSI within 5 minutes, and the diagram of the waveform 2 corresponding to the change period of the phase of the third CSI within 5 minutes. In FIG. 9C, a time interval between a wave peak 1 and a wave peak 2 of the waveform 1 is T1, and a time interval between a wave peak 2 and a wave peak 3 of the waveform 1 is T2. A time interval between a wave trough 1 and a wave trough 2 of the waveform 2 is T3, and a time interval between a wave trough 2 and a wave trough 3 of the waveform 2 is T4. In this case, the first node 101 may obtain an average time interval (T1+T2+T3+T4)/4, and use (T1+T2+T3+T4)/4 as a time interval between two times of breathing of the user. In this way, the first node 101 may determine the breathing frequency of the user based on the time interval.

Example 2: The sensing feature of the target is a posture of the user kicking in front of a trunk of a vehicle.

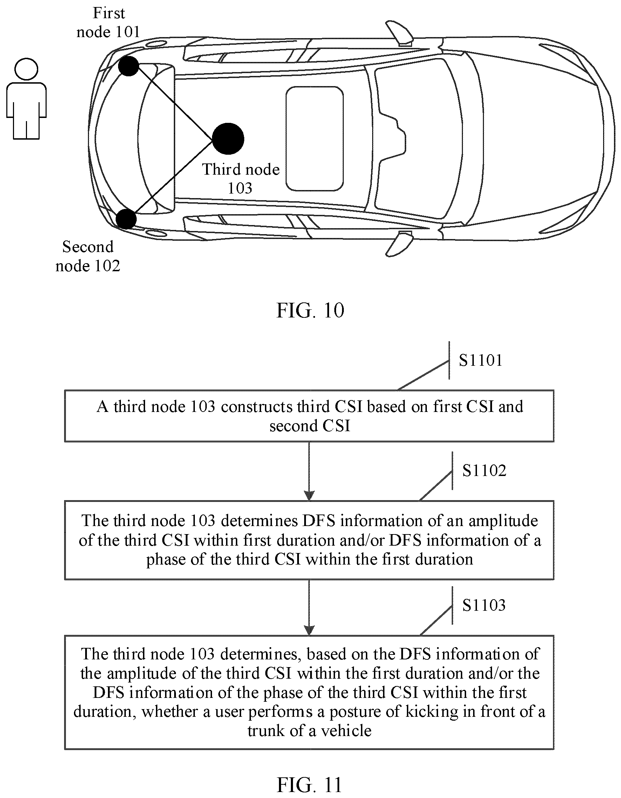

FIG. 10 is a diagram 1 of a scenario in which non-contact sensing is applied to a vehicle. The scenario includes a vehicle and a user. A first node 101, a second node 102, and a third node 103 are deployed on the vehicle. The first node 101 and the second node 102 may be, for example, vehicle-mounted cameras with specific communication functions, and the third node 103 may be, for example, a vehicle-mounted controller.

The first node 101, the second node 102, and the third node 103 may exchange information through an in-vehicle communication network. For example, the first node 101 may send a sensing signal 1 to the second node 102, and the second node 102 may determine first CSI based on the sensing signal 1, and send the first CSI to the third node 103. The second node 102 may send a sensing signal 2 to the first node 101, and then the first node 101 may determine second CSI based on the sensing signal 2, and send the second CSI to the third node 103. Because the first CSI and the second CSI change with a body posture of the user, the third node 103 may detect, based on the first CSI and the second CSI, whether the user performs a posture of kicking in front of a trunk of the vehicle. If the user performs the posture, the third node 103 may control a rear trunk door of the vehicle to automatically open.

As shown in FIG. 11, a specific procedure in which the third node 103 detects, based on the first CSI and the second CSI, whether the user performs the posture of kicking in front of the trunk of the vehicle includes the following steps.

S1101: The third node 103 constructs third CSI based on the first CSI and the second CSI.

In S1101, the third node 103 constructs the third CSI based on the first CSI and the second CSI, so that impact of phase offsets and/or amplitude offsets of the first CSI and the second CSI on a user posture detection process can be eliminated.

S1102: The third node 103 determines DFS information of an amplitude of the third CSI within first duration and/or DFS information of a phase of the third CSI within the first duration.

S1103: The third node 103 determines, based on the DFS information of the amplitude of the third CSI within the first duration and/or the DFS information of the phase of the third CSI within the first duration, whether the user performs the posture of kicking in front of the trunk of the vehicle.

It should be understood that different DFS information corresponds to different body postures of the user.

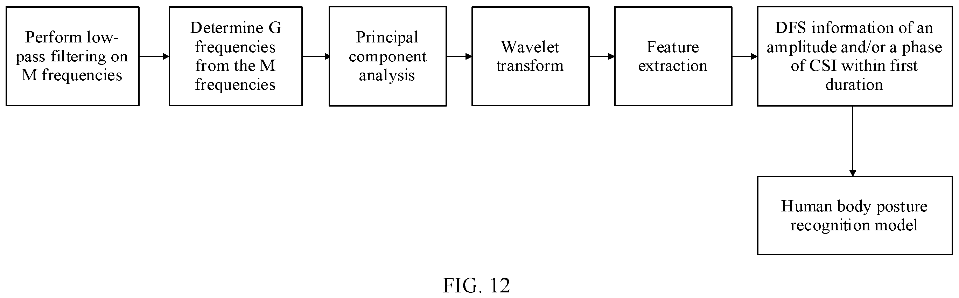

As shown in FIG. 12, in a possible implementation, the third CSI includes CSI corresponding to M frequencies. First, the third node 103 may perform low-pass filtering on the CSI corresponding to the M frequencies. Then, the third node 103 may determine variances corresponding to the filtered CSI corresponding to the M frequencies, determine G frequencies that are in the M frequencies and whose variances corresponding to the CSI are greater than a first threshold, and sequentially perform operations such as principal component analysis, wavelet transform, and feature extraction on the CSI corresponding to the G frequencies, to obtain DFS information of amplitudes and/or phases of the CSI corresponding to the G frequencies within the first duration. The third node 103 determines a current human body posture feature of the user based on the DFS information of the amplitudes and/or phases of the CSI corresponding to the G frequencies within the first duration. Further, the third node 103 inputs the current human body posture feature of the user into a human body posture recognition model. If a human body posture output by the human body posture recognition model is a posture of the user kicking in front of the trunk of the vehicle, the third node 103 controls the rear trunk door of the vehicle to automatically open. The human body posture recognition model may be, for example, a support vector machine (support vector machine, SVM) classifier.

Based on the foregoing method embodiments, an embodiment of this application further provides a non-contact sensing apparatus, configured to perform the method in the foregoing method embodiments. For related features, refer to the foregoing method embodiments. Details are not described herein again.

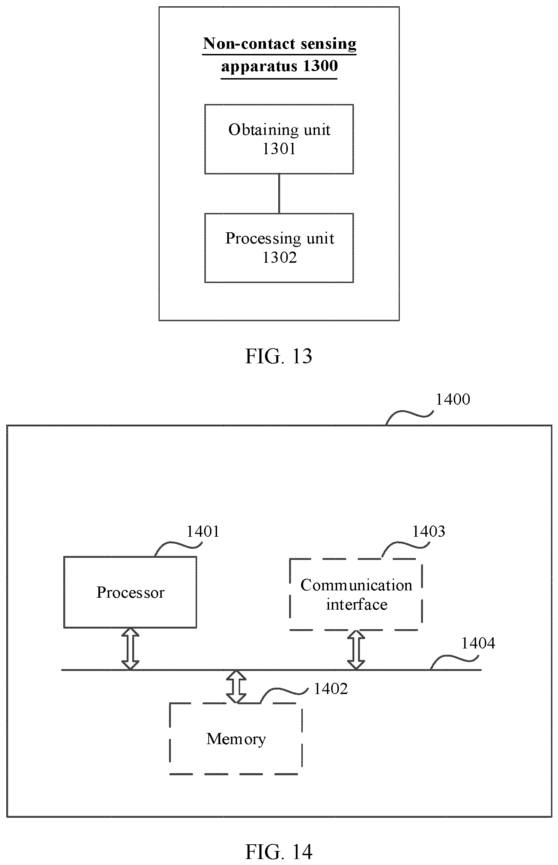

As shown in FIG. 13, the non-contact sensing apparatus 1300 may include: an obtaining unit 1301, configured to obtain first information, where the first information is associated with first channel state information CSI and second CSI of a first frequency, the first CSI is CSI of a link from a first node to a second node, and the second CSI is CSI of a link from the second node to the first node; and a processing unit 1302, configured to determine a sensing feature of a target based on the first information.

In a possible design, when being configured to determine the sensing feature of the target based on the first information, the processing unit 1302 is specifically configured to: construct third CSI based on the first CSI and the second CSI; and determine the sensing feature based on the third CSI.

In a possible design, that the processing unit 1302 constructs the third CSI based on the first CSI and the second CSI includes: performing multiplication or conjugate multiplication on the first CSI and the second CSI, to obtain the third CSI.

In a possible design, the apparatus is applied to the first node, and that the obtaining unit 1301 obtains the first CSI and the second CSI includes: receiving the first CSI from the second node, where the first CSI is associated with a first sensing signal sent by the first node; and receiving a second sensing signal from the second node, and determining second CSI based on the second sensing signal.

In a possible design, the apparatus is applied to the second node, and that the obtaining unit 1301 obtains the first CSI and the second CSI of the first frequency includes: receiving a first sensing signal from the first node, and determining the first CSI based on the first sensing signal; and receiving the second CSI from the first node, where the second CSI is associated with a second sensing signal sent by the second node.

In a possible design, the apparatus is applied to a third node, and that the obtaining unit 1301 obtains the first channel state information CSI and the second CSI of the first frequency includes: receiving the first CSI from the second node, where the first CSI is associated with a first sensing signal sent by the first node; and receiving the second CSI from the first node, where the second CSI is associated with a second sensing signal sent by the second node.

In a possible design, that the processing unit 1302 determines the sensing feature based on the third CSI includes: extracting an amplitude and/or a phase corresponding to the third CSI; determining a statistical feature of the amplitude and/or a statistical feature of the phase, where the statistical feature of the amplitude includes at least one of a change period, a power spectral density variance, Doppler frequency shift DFS information, or the like of the amplitude within first duration, and the statistical feature of the phase includes at least one of a change period, a power spectral density variance, DFS information, or the like of the phase within the first duration; and determining the sensing feature based on the statistical feature of the amplitude and/or the statistical feature of the phase.

In a possible design, that the processing unit 1302 determines the sensing feature based on the statistical feature of the amplitude and/or the statistical feature of the phase includes: determining the sensing feature based on the statistical feature of the amplitude when the power spectral density variance of the amplitude is greater than the power spectral density variance of the phase; or determining the sensing feature based on the statistical feature of the phase when the power spectral density variance of the amplitude is less than the power spectral density variance of the phase.

In a possible design, when the sensing feature is breathing frequency, that the processing unit 1302 determines the sensing feature based on the statistical feature of the amplitude and/or the statistical feature of the phase includes: converting the change period of the amplitude within the first duration and/or the change period of the phase within the first duration into a first waveform; calculating an average time interval between k wave peaks or k wave troughs in the first waveform, where the average time interval represents a time interval between two times of breathing of a user; and determining the breathing frequency based on the average time interval.

In a possible design, when the sensing feature is a human body posture, that the processing unit 1302 determines the sensing feature based on the statistical feature of the amplitude and/or the statistical feature of the phase includes: inputting the DFS information of the amplitude within the first duration and/or the DFS information of the phase within the first duration into a classifier, to obtain the human body posture.

In a possible design, the first CSI is N pieces of CSI, the second CSI is N pieces of CSI, and the third CSI includes N pieces of CSI.

In a possible design, the power spectral density variance of the amplitude of the third CSI corresponding to the first frequency is greater than a first threshold, and the power spectral density variance of the phase of the third CSI corresponding to the first frequency is greater than a second threshold.

In a possible design, the first frequency is one of M frequencies, and the obtaining unit 1301 is further configured to: obtain first CSI and second CSI that correspond to a frequency other than the first frequency in the M frequencies, where the power spectral density variance of the amplitude of the third CSI corresponding to the first frequency is greater than a power spectral density variance of an amplitude of third CSI corresponding to the another frequency, and the power spectral density variance of the phase of the third CSI corresponding to the first frequency is greater than a power spectral density variance of a phase of the third CSI corresponding to the another frequency.

In a possible design, the first information is third CSI or a temporary sensing feature of the target, and the third CSI or the temporary sensing feature of the target is associated with the first CSI and the second CSI.

In a possible design, when the apparatus is applied to a fourth node, the obtaining unit 1301 is configured to receive the third CSI from the second node or the first node; or the obtaining unit 1301 is configured to receive the temporary sensing feature of the target from the second node or the first node.

For a specific implementation, refer to the method steps implemented in the foregoing method embodiments. Details are not described herein again.

It should be understood that division of units in the apparatus is merely logical function division. During actual implementation, all or some of the units may be integrated into one physical entity or may be physically separated. In addition, the units in the apparatus may be implemented in a form of software invoked by a processor. For example, the apparatus includes a processor. The processor is connected to a memory. The memory stores instructions. The processor invokes the instructions stored in the memory, to implement any one of the foregoing methods or implement functions of each unit of the apparatus. The processor is, for example, a general-purpose processor, for example, a central processing unit (Central Processing Unit, CPU) or a microprocessor. The memory is a memory inside the apparatus or a memory outside the apparatus. Alternatively, the units in the apparatus may be implemented in a form of hardware circuits, and functions of some or all of units may be implemented by designing the hardware circuits. The hardware circuits may be understood as one or more processors. For example, in an implementation, the hardware circuit is an application-specific integrated circuit (application-specific integrated circuit, ASIC), and the functions of some or all of the foregoing units are implemented by designing a logical relationship between elements in the circuit. For another example, in another implementation, the hardware circuit may be implemented by using a programmable logic device (programmable logic device, PLD). A field programmable gate array (Field Programmable Gate Array, FPGA) is used as an example, and the field programmable gate array may include a large quantity of logic gate circuits. A configuration file is used to configure a connection relationship between logic gate circuits, to implement functions of some or all of the foregoing units. All units of the foregoing apparatus may be implemented in a form of software invoked by the processor, or all units may be implemented in a form of hardware circuit, or some units may be implemented in a form of software invoked by the processor, and a remaining part may be implemented in a form of hardware circuit.

In embodiments of this application, the processor is a circuit with a signal processing capability. In an implementation, the processor may be a circuit with an instruction reading and running capability, for example, a CPU, a microprocessor, a graphics processing unit (graphics processing unit, GPU) (which may be understood as a microprocessor), or a digital signal processor (digital signal processor, DSP). In another implementation, the processor may implement a specific function based on a logical relationship of a hardware circuit. The logical relationship of the hardware circuit is fixed or reconfigurable. For example, the processor is a hardware circuit implemented by an ASIC or a PLD, for example, an FPGA. In the reconfigurable hardware circuit, a process in which the processor loads a configuration document to implement hardware circuit configuration may be understood as a process in which the processor loads instructions to implement functions of some or all of the foregoing units. In addition, the processor may alternatively be a hardware circuit designed for artificial intelligence, and may be understood as the ASIC, for example, a neural network processing unit (Neural Network Processing Unit, NPU), a tensor processing unit (Tensor Processing Unit, TPU), or a deep learning processing unit (Deep learning Processing Unit, DPU).

It can be learned that each unit of the foregoing apparatus may be one or more processors (or processing circuits) configured to implement the foregoing method, for example, a CPU, a GPU, an NPU, a TPU, a DPU, a microprocessor, a DSP, an ASIC, or an FPGA, or a combination of at least two of these processor forms.

In addition, all or some of the units of the foregoing apparatus may be integrated, or may be implemented independently. In an implementation, these units are integrated together and implemented in a form of system-on-a-chip (system-on-a-chip, SOC). The SOC may include at least one processor, configured to implement any one of the methods or implement functions of the units of the apparatus. Types of the at least one processor may be different. For example, the at least one processor includes a CPU and an FPGA, a CPU and an artificial intelligence processor, or a CPU and a GPU.

An embodiment of this application further relates to a chip system. The chip system 1400 includes at least one processor 1401 and a communication interface 1403. In an optional design, the chip system 1400 may further include a memory 1402.

In embodiments of this application, a specific connection medium between the processor 1401 and the memory 1402 is not limited. For example, the connection medium may be a bus 1404.

In the chip system 1400, when communicating with another device, the processor 1401 may perform data transmission through the communication interface 1403. The processor 1401 in FIG. 14 may invoke computer-executable instructions stored in the memory 1402, so that the chip system 1400 can perform any one of the foregoing method embodiments.

The processor is configured to invoke a computer program or computer instructions stored in the memory, so that the processor performs the method according to any one of the foregoing embodiments.

In a possible implementation, the processor is coupled to the memory through an interface.

In a possible implementation, the chip system may alternatively directly include a memory. The memory stores a computer program or computer instructions.

For example, the memory may be a volatile memory or a nonvolatile memory, or may include both a volatile memory and a nonvolatile memory. The nonvolatile memory may be a read-only memory (read-only memory, ROM), a programmable read-only memory (programmable ROM, PROM), or an erasable programmable read-only memory (erasable PROM, EPROM), an electrically erasable programmable read-only memory (electrically EPROM, EEPROM), or a flash memory. The volatile memory may be a random access memory (random access memory, RAM) and is used as an external cache. Through example but not limitative descriptions, many forms of RAMs may be used, for example, a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM).

An embodiment of this application further relates to a processor. The processor is configured to invoke a computer program or computer instructions stored in a memory, so that the processor performs the method according to any one of the foregoing embodiments.

In an example, in embodiments of this application, the processor is an integrated circuit chip, and has a signal processing capability. For example, the processor may be an FPGA, may be a general-purpose processor, a DSP, an ASIC or another programmable logic device, a discrete gate or a transistor logic device, or a discrete hardware component, or may be a system-on-a-chip (system-on-a-chip, SoC), or may be a CPU, or may be a network processor (network processor, NP), or may be a microcontroller unit (microcontroller unit, MCU), or may be a PLD or another integrated chip, and may implement or perform the methods, steps, and logical block diagrams disclosed in embodiments of this application. The general-purpose processor may be a microprocessor, or the processor may be any conventional processor or the like. The steps in the methods disclosed with reference to embodiments of this application may be directly performed and completed by a hardware decoding processor, or may be performed and completed by using a combination of hardware in the decoding processor and a software module. The software module may be located in a mature storage medium in the art, for example, a random access memory, a flash memory, a read-only memory, a programmable read-only memory, an electrically erasable programmable memory, or a register. The storage medium is located in the memory, and the processor reads information in the memory and completes the steps in the foregoing methods in combination with hardware of the processor.

It should be understood that embodiments of this application may be provided as a method, a system, or a computer program product.

In a possible implementation, an embodiment of this application provides a computer-readable storage medium. The computer-readable storage medium stores program code, and when the program code is run on a computer, the computer is enabled to perform the foregoing method embodiments.

In a possible implementation, an embodiment of this application provides a computer program product. When the computer program product runs on a computer, the computer is enabled to perform the foregoing method embodiments.

Therefore, this application may use a form of hardware only embodiments, software only embodiments, or embodiments with a combination of software and hardware. In addition, this application may use a form of computer program product that is implemented on one or more computer-usable storage media (including but not limited to a disk memory, a CD-ROM, an optical memory, and the like) that include computer-usable program code.

These computer program instructions may alternatively be stored in a computer-readable memory that can indicate the computer or the another programmable data processing device to work in a specific manner, so that the instructions stored in the computer-readable memory generate an artifact that includes an instruction apparatus. The instruction apparatus implements a specific function in one or more processes in the flowcharts and/or in one or more blocks in the block diagrams.

The computer program instructions may alternatively be loaded onto a computer or another programmable data processing device, so that a series of operations and steps are performed on the computer or the another programmable device, to generate computer-implemented processing. Therefore, the instructions executed on the computer or the another programmable device provide steps for implementing a specific function in one or more procedures in the flowcharts and/or in one or more blocks in the block diagrams.

It is clearly that a person skilled in the art can make various modifications and variations to embodiments of this application without departing from the scope of embodiments of this application. In this case, this application is intended to cover these modifications and variations of the embodiments of this application provided that they fall within the scope of protection defined by the following claims and their equivalent technologies. In embodiments of this application, unless otherwise stated or there is a logic conflict, terms and/or descriptions between embodiments are consistent and may be mutually referenced, and technical features in different embodiments may be combined based on an internal logical relationship thereof, to form a new embodiment.

## Claims

1. A non-contact sensing method, comprising:
obtaining first information, wherein the first information is associated with first channel state information CSI and second CSI of a first frequency, the first CSI is CSI of a link from a first node to a second node, and the second CSI is CSI of a link from the second node and the first node; and
determining a sensing feature of a target based on the first information.

2. The method according to claim 1, wherein the first information is the first CSI and the second CSI that correspond to the first frequency.

3. The method according to claim 2, wherein determining the sensing feature of the target based on the first information comprises:
constructing third CSI based on the first CSI and the second CSI; and
determining the sensing feature based on the third CSI.

4. The method according to claim 3, wherein constructing the third CSI based on the first CSI and the second CSI comprises:
performing a first operation on the first CSI and the second CSI to obtain the third CSI, wherein
the first operation is any one of the following: performing multiplication on the first CSI and the second CSI, performing conjugate multiplication on the first CSI and the second CSI, performing division on the first CSI and the second CSI, or performing conjugate division on the first CSI and the second CSI.

5. The method according to any one of claims 2 to 4, wherein the method is applied to the first node, and obtaining the first CSI and the second CSI comprises:
receiving the first CSI from the second node, wherein the first CSI is associated with a first sensing signal sent by the first node; and
receiving a second sensing signal from the second node, and determining the second CSI based on the second sensing signal.

6. The method according to any one of claims 2 to 4, wherein the method is applied to the second node, and obtaining the first CSI and the second CSI of the first frequency comprises:
receiving a first sensing signal from the first node, and determining the first CSI based on the first sensing signal; and
receiving the second CSI from the first node, wherein the second CSI is associated with a second sensing signal sent by the second node.

7. The method according to any one of claims 2 to 4, wherein the method is applied to a third node, and obtaining the first channel state information CSI and the second CSI of the first frequency comprises:
receiving the first CSI from the second node, wherein the first CSI is associated with a first sensing signal sent by the first node; and
receiving the second CSI from the first node, wherein the second CSI is associated with a second sensing signal sent by the second node.

8. The method according to any one of claims 3 to 7, wherein determining the sensing feature based on the third CSI comprises:
extracting an amplitude and/or a phase corresponding to the third CSI;
determining a statistical feature of the amplitude and/or a statistical feature of the phase, wherein the statistical feature of the amplitude comprises at least one of a change period, a power spectral density variance, Doppler frequency shift DFS information, or the like of the amplitude within first duration, and the statistical feature of the phase comprises at least one of a change period, a power spectral density variance, DFS information, or the like of the phase within the first duration; and
determining the sensing feature based on the statistical feature of the amplitude and/or the statistical feature of the phase.

9. The method according to claim 8, wherein determining the sensing feature based on the statistical feature of the amplitude and/or the statistical feature of the phase comprises:
determining the sensing feature based on the statistical feature of the amplitude when the power spectral density variance of the amplitude is greater than the power spectral density variance of the phase; or determining the sensing feature based on the statistical feature of the phase when the power spectral density variance of the amplitude is less than the power spectral density variance of the phase.

10. The method according to claim 8 or 9, wherein when the sensing feature is breathing frequency, determining the sensing feature based on the statistical feature of the amplitude and/or the statistical feature of the phase comprises:
converting the change period of the amplitude within the first duration and/or the change period of the phase within the first duration into a first waveform;
calculating an average time interval between k wave peaks or k wave troughs in the first waveform, wherein the average time interval represents a time interval between two times of breathing of a user; and
determining the breathing frequency based on the average time interval.

11. The method according to claim 8 or 9, wherein when the sensing feature is a human body posture, determining the sensing feature based on the statistical feature of the amplitude and/or the statistical feature of the phase comprises:
inputting the DFS information of the amplitude within the first duration and/or the DFS information of the phase within the first duration into a classifier, to obtain the human body posture.

12. The method according to any one of claims 3, 4, and 8 to 11, wherein the first CSI is N pieces of CSI, the second CSI is N pieces of CSI, and the third CSI comprises N pieces of CSI.

13. The method according to any one of claims 3, 4, and 8 to 12, wherein the power spectral density variance of the amplitude of the third CSI corresponding to the first frequency is greater than a first threshold, and/or the power spectral density variance of the phase of the third CSI corresponding to the first frequency is greater than a second threshold.

14. The method according to any one of claims 3, 4, and 8 to 13, wherein the first frequency is one of M frequencies, and the method further comprises:
obtaining first CSI and second CSI that correspond to a frequency other than the first frequency in the M frequencies, wherein
the power spectral density variance of the amplitude of third CSI corresponding to the first frequency is greater than a power spectral density variance of an amplitude of the third CSI corresponding to the another frequency, and the power spectral density variance of the phase of the third CSI corresponding to the first frequency is greater than a power spectral density variance of a phase of the third CSI corresponding to the another frequency.

15. The method according to claim 1, wherein the first information is third CSI or a temporary sensing feature of the target, and the third CSI or the temporary sensing feature of the target is associated with the first CSI and the second CSI.

16. The method according to claim 15, wherein the method is applied to a fourth node; and
obtaining the third CSI comprises: receiving the third CSI from the second node or the first node; or
obtaining the temporary sensing feature of the target comprises: receiving the temporary sensing feature of the target from the second node or the first node.

17. The method according to any one of claims 2 to 16, wherein the first CSI and the second CSI correspond to a same antenna port pair.

18. A non-contact sensing apparatus, comprising:
an obtaining unit, configured to obtain first information, wherein the first information is associated with first channel state information CSI and second CSI of a first frequency, the first CSI is CSI of a link from a first node to a second node, and the second CSI is CSI of a link from the second node and the first node; and
a processing unit, configured to determine a sensing feature of a target based on the first information.

19. The apparatus according to claim 18, wherein the first information is the first CSI and the second CSI that correspond to the first frequency.

20. The apparatus according to claim 19, wherein when determining the sensing feature of the target based on the first information, the processing unit is specifically configured to:
construct third CSI based on the first CSI and the second CSI; and
determine the sensing feature based on the third CSI.

21. The apparatus according to claim 20, wherein that the processing unit constructs the third CSI based on the first CSI and the second CSI comprises:
performing a first operation on the first CSI and the second CSI to obtain the third CSI, wherein
the first operation is any one of the following: performing multiplication on the first CSI and the second CSI, performing conjugate multiplication on the first CSI and the second CSI, performing division on the first CSI and the second CSI, or performing conjugate division on the first CSI and the second CSI.

22. The apparatus according to any one of claims 19 to 21, wherein the apparatus is used in the first node, and that the obtaining unit obtains the first CSI and the second CSI comprises:
receiving the first CSI from the second node, wherein the first CSI is associated with a first sensing signal sent by the first node; and
receiving a second sensing signal from the second node, and determining the second CSI based on the second sensing signal.

23. The apparatus according to any one of claims 19 to 21, wherein the apparatus is used in the second node, and the obtaining unit obtains the first CSI and the second CSI of the first frequency comprises:
receiving a first sensing signal from the first node, and determining the first CSI based on the first sensing signal; and
receiving the second CSI from the first node, wherein the second CSI is associated with a second sensing signal sent by the second node.

24. The apparatus according to any one of claims 19 to 21, wherein the apparatus is used in a third node, and the obtaining unit obtains the first channel state information CSI and the second CSI of the first frequency comprises:
receiving the first CSI from the second node, wherein the first CSI is associated with a first sensing signal sent by the first node; and
receiving the second CSI from the first node, wherein the second CSI is associated with a second sensing signal sent by the second node.

25. The apparatus according to any one of claims 20 to 24, wherein that the processing unit determines the sensing feature based on the third CSI comprises:
extracting an amplitude and/or a phase corresponding to the third CSI;
determining a statistical feature of the amplitude and/or a statistical feature of the phase, wherein the statistical feature of the amplitude comprises at least one of a change period, a power spectral density variance, or Doppler frequency shift DFS information of the amplitude within first duration, and the statistical feature of the phase comprises at least one of a change period, a power spectral density variance, or DFS information of the phase within the first duration; and
determining the sensing feature based on the statistical feature of the amplitude and/or the statistical feature of the phase.

26. The apparatus according to claim 25, wherein that the processing unit determines the sensing feature based on the statistical feature of the amplitude and/or the statistical feature of the phase comprises:
determining the sensing feature based on the statistical feature of the amplitude when the power spectral density variance of the amplitude is greater than the power spectral density variance of the phase; or determining the sensing feature based on the statistical feature of the phase when the power spectral density variance of the amplitude is less than the power spectral density variance of the phase.

27. The apparatus according to claim 25 or 26, wherein when the sensing feature is breathing frequency, that the processing unit determines the sensing feature based on the statistical feature of the amplitude and/or the statistical feature of the phase comprises:
converting the change period of the amplitude within the first duration and/or the change period of the phase within the first duration into a first waveform;
calculating an average time interval between k wave peaks or k wave troughs in the first waveform, wherein the average time interval represents a time interval between two times of breathing of a user; and
determining the breathing frequency based on the average time interval.

28. The apparatus according to claim 25 or 26, wherein when the sensing feature is a human body posture, that the processing unit determines the sensing feature based on the statistical feature of the amplitude and/or the statistical feature of the phase comprises:
inputting the DFS information of the amplitude within the first duration and/or the DFS information of the phase within the first duration into a classifier, to obtain the human body posture.

29. The apparatus according to any one of claims 20, 21, and 25 to 28, wherein the first CSI is N pieces of CSI, the second CSI is N pieces of CSI, and the third CSI comprises N pieces of CSI.

30. The apparatus according to any one of claims 20, 21, and 25 to 29, wherein the power spectral density variance of the amplitude of the third CSI corresponding to the first frequency is greater than a first threshold, and/or the power spectral density variance of the phase of the third CSI corresponding to the first frequency is greater than a second threshold.

31. The apparatus according to any one of claims 20, 21, and 25 to 30, wherein the first frequency is one of M frequencies, and the obtaining unit is further configured to:
obtain first CSI and second CSI that correspond to a frequency other than the first frequency in the M frequencies, wherein
the power spectral density variance of the amplitude of third CSI corresponding to the first frequency is greater than a power spectral density variance of an amplitude of the third CSI corresponding to the another frequency, and the power spectral density variance of the phase of the third CSI corresponding to the first frequency is greater than a power spectral density variance of a phase of the third CSI corresponding to the another frequency.

32. The apparatus according to claim 18, wherein the first information is third CSI or a temporary sensing feature of the target, and the third CSI or the temporary sensing feature of the target is associated with the first CSI and the second CSI.

33. The apparatus according to claim 32, wherein the apparatus is used in a fourth node; and
obtaining the third CSI comprises: receiving the third CSI from the second node or the first node; or
obtaining the temporary sensing feature of the target comprises: receiving the temporary sensing feature of the target from the second node or the first node.

34. The apparatus according to any one of claims 19 to 33, wherein the first CSI and the second CSI correspond to a same antenna port pair.

35. A computer-readable storage medium, wherein the storage medium stores a computer program or instructions, and when the computer program or the instructions are executed by a communication apparatus, the method according to any one of claims 1 to 17 is implemented.

36. A communication apparatus, comprising at least one processor and an interface circuit, wherein the interface circuit is configured to: receive a signal from a communication apparatus other than the communication apparatus and transmit the signal to the processor, or send a signal from the processor to a communication apparatus other than the communication apparatus; and the processor is configured to implement the method according to any one of claims 1 to 17 by using a logic circuit or by executing code instructions.

37. A terminal, wherein the terminal comprises the apparatus according to any one of claims 18 to 34.

38. The terminal according to claim 37, wherein the terminal is a vehicle.
